# EUROPÄISCHE PATENTANMELDUNG

(11) **EP 2 249 157 A1**
(43) Veröffentlichungstag der Anmeldung: **10.11.2010**
(21) Anmeldenummer: 10173987.8
(22) Anmeldetag: 07.11.2007
(51) Int. Cl.: G01N 33/543, C12N 5/00

(54) **Verfahren zur Aufreinigung wenigstens einer nachzuweisenden Zielsubstanz**

(30) Priorität: 08.11.2006 DE 102006052923; 21.12.2006 DE 102006060717
(62) Teilanmeldung aus: 07819658.1
(71) Anmelder: Peter, Jochen, 73732 Esslingen a. N. (DE)
(72) Erfinder: Peter, Jochen, 73732 Esslingen a. N. (DE)
(74) Vertreter: Reinhard - Skuhra - Weise & Partner GbR

(57) **Zusammenfassung**

Beschrieben wird ein Produkt, mit Magnetbeads, die eine funktionalisierte Oberfläche aufweisen und in folgender Weise herstellbar sind: Bereitstellen von magnetischen Partikeln, kurz Beads, mit einer Partikelgröße im Nano- und/oder Mikro-Bereich, d.h. ein oder mehrere nm oder µm, und mit an deren Oberflächen applizierte funktionelle Gruppe, wie z.B. Aminogruppen; Reagieren der Beads in einem Verzweigungsreagenz, wie z.B. Methylacrylat enthaltenden organischen Lösungsmittel; Abtrennen der Beads aus dem organischen Lösungsmittel und Waschen der Beads mit einem organischen Lösungsmittel; Reagieren der Beads mit einem Verbinder, wie z.B. Ethylendiamin, enthaltenden organischen Flüssigkeit; Abtrennen der Beads aus der organischen Flüssigkeit und Waschen der Beads; n - maliges Wiederholen der Schritte b) bis e); Suspendieren der Beads in einer mit wenigstens einem Derivat enthaltenden Lösung, und Abtrennen und Waschen der Beads und Bereitstellen der derivatisierten Beads in Wasser oder pufferhaltiger Lösung.

## Beschreibung

### Technisches Gebiet

Die Erfindung bezieht sich auf ein Verfahren zur Aufreinigung wenigstens einer nachzuweisenden Zielsubstanz, die während einer Zellkultivierung in einem Zellkulturmedium auftritt oder gebildet wird, bei dem zu dem Zellkulturmedium magnetische, an ihrer Oberfläche funktionalisierte Partikel, so genannte Beads, zugegeben werden, an deren Oberfläche sich die Zielsubstanz selektiv anlagert und durch Applikation eines Magnetfeldes die mit der Zielsubstanz behafteten Partikel aus dem Zellkulturmedium selektiert werden.

### Stand der Technik

Gattungsgemäße Verfahren werden bevorzugt zur präparativen Isolierung von Substanzen, welche von Zellen in ein Zellkulturmedium abgegeben werden eingesetzt, um Zelleigenschaften sowie auch ihr weiteres Wachstumsverhalten studieren zu können. Von besonderem Interesse ist dabei die Erforschung von Tumorzellen, deren unkontrolliertes Zellwachstum verantwortlich für die häufigste Todesursache beim Menschen ist.

Für eine verantwortungsvolle Therapie zur Bekämpfung eines beim Menschen festgestellten Tumors ist es unablässig zunächst die Tumorart zu bestimmen, bspw. handelt es sich um eine nicht invasive, invasive Tumorart oder um ductales Carcinoma in situ, etc.. Die Art des Tumors ist hierbei entscheidend für den weiteren Verlauf der Therapie. Hintergrund dabei ist, dass Tumorzellen im Laufe der Progression ihre Eigenschaften verändern. So besitzt bspw. die nicht invasive Krebszelllinie MCF-7 einen Östrogenrezeptor, so dass diese Art von Tumore mit einem Inhibitor des Östrogenrezeptors, z.B. mit Tamixofen, behandelt werden können. Im Wege der Progression kann eine Umwandlung einer gutartigen, d.h. nicht invasiven Tumorzelle in einen bösartigen, d.h. invasiven, Zelltyp erfolgen, bei dem der Östrogenrezeptor an der Zelloberfläche nicht mehr präsentiert wird und daher eine Hormonhandlung nicht mehr möglich ist.

Grundsätzlich wird die Progression eines Tumors durch Mutationen ausgelöst, die wiederum durch äußere Einflüsse wie z.B. Sauerstoffarmut (Hypoxie) hervorgerufen werden können. Neben der Zelleigenschaft, dass Tumorzellen nicht nur Rezeptoren an ihrer Oberfläche vorsehen, die in Art, Wirkung und Anzahl aufgrund der Mutation starken Veränderungen unterliegen, verändern Tumorzellen aber auch ihr Verhalten in der Sekretion von verschiedenen regulatorischen Peptiden und Proteinen.

Ein Ansatz zur Findung von Tumormarkern aus Serum/Plasma heraus wird in der modernen Proteomforschung unter der Verwendung verschiedener chromatographischer Schritte, der Verwendung von 2-D-Gelelektrophorese und anschließender massenspektrometrischer Methoden durchgeführt. Gegenwärtig ist jedoch aufgrund von sehr hoch konzenrtierten Proteinen im Serum/Plasma die Suche nach potentiellen Markern durch Überlagerungseffekte ins Stocken geraten. Aus diesem Grunde werden Untersuchungen mit Tumorzelllinien durchgeführt. Derartige Untersuchungen an Tumorzellen haben aber gezeigt, dass Peptide und Proteine nur in äußerst geringen Konzentrationen, d.h. Stoffmengen von 10⁻¹² bis 10⁻⁹ M, von Zellen abgegeben werden. Da das Detektionslimit der gängigen Massenspektrometern zur Zeit bei 10⁻⁹ M liegt und für die massenspektrometrische Detektion die Substanzen hochrein vorliegen müssen, sind für die Anreicherung von Analyten in Zellkulturüberständen, z.B. Sekretionsmolekülen, effiziente Aufreinigungsschritte unabdingbar.

Aus diesem Grund liegt der Hauptaugenmerk der Proteomforschung momentan in der Präanalytik von niedrigkonzentrierten Substanzen. Eine Serie von verschieden aufeinander folgenden Aufreinigungsschritten führt aber bei einer geringen Proteinmenge durch unspezifische Bindungen an den zu verwendeten Gefäßwänden zu einem totalen Verlust an Protein. Ein zusätzliches Problem bei vielen Aufreinigungsschritten ergibt sich durch eine zunehmende Kontamination der Proben, die eine massenspektrometrische Analyse durch Störsignale unmöglich machen kann.

Deshalb konnten sekretorische Untersuchungen mit Zellen bisher nur mit sehr hohen Zellzahlen durchgeführt werden.

Experimente haben gezeigt, dass bei einer anfänglichen Zellzahl in der Größenordnung von 10⁷ Zellen und einer mehrtägigen Vermehrungsphase in einem serumhaltigen Medium und anschließender Inkubation in serumfreier Umgebung lediglich wenige mg Gesamtproteine erhalten werden. Bspw. wurde bei der Untersuchung von Hefezellen nach Abschluss der Zellkultur bei einer Zellzahl von 10⁹ Zellen eine Konzentration von 30 nM AIP-Protein in den Überständen gemessen. Solch große Mengen an Zellen stehen aber in der Regel nicht zur Verfügung. Bei der Untersuchung von Krebszellen, die aus Tumorgewebe durch Biopsie entnommen werden, können pro Stechzylinder maximal einige 10⁵ Zellen isoliert werden, wobei viele Zellen davon nicht Tumorzellen sind. Aufgrund der sehr geringen Konzentration der ausgeschütteten Substanzen ist es somit äußerst wichtig, die Sekretionsmoleküle in einem einzigen Aufreinigungsschritt effizient zu isolieren und der nachfolgenden Analytik ohne Umwege zuzuführen.

In den letzten 5 Jahren wurde versucht, als Screeningmethode für die von Zellen sekretierten Peptide und Proteine die SELDI-TOF-MS Technologie (Surface Enhanced Laser Desorption lonization Time of Flight Mass Spectrometry) einzusetzen. Hierzu werden auf einem Chip mit einer meist hydrophoben Oberfläche von der Größe von 3 mm² die zu untersuchenden Proben aufgegeben. Die auf dem Chip gebundenen Proteine werden anschließend durch Laserbeschuss ionisiert, massenspektrometrisch detektiert und in einem 2-dimensionalen Plot graphisch dargestellt. Durch die Anwendung dieser Methode konnten z.B. verschiedene Krebszelllinien miteinander verglichen werden. Ein limitierender Faktor ist jedoch die ungenügende Empfindlichkeit dieser Technologie. In der Regel liegt die Empfindlichkeitsgrenze für dieses System bei maximal 300 nM und aus diesem Grund wird die SELDI-TOF-MS-Technologie meist nur noch für das Profiling von Serumproben eingesetzt.

Eine Steigerung der Empfindlichkeit kann nur durch den Einsatz von Partikeln mit geringer Größe erfolgen, da dadurch eine größere Gesamtoberfläche für die Bindung von Protein zur Verfügung steht. Durch die Verwendung von magnetischen Partikeln als Trägersubstanzen für die selektierenden Proteine und Peptide ist eine Automatisierung und somit ein höherer Durchsatz an Proben möglich. Der Einsatz von magnetischen Partikeln, so genannte Beads, zur Isolierung von Proteinen wurde mit biotinylierten Antikörpern, welche an magnetische Streptavidinbeads gekoppelt waren, gezeigt und erfolgreich für die massenspektrometrische Untersuchung von Prostata Spezifischen Antigen (PSA) aus Seren heraus verwendet. Leider kann aber durch den Einsatz von magnetischen "Antikörper-Partikeln" jeweils nur ein einzelnes Protein isoliert werden.

Für die Bindung von möglichst vielen unterschiedlichen Peptiden oder Proteinen aus einer Lösung, wie Plasma, Serum, Urin oder Zellkulturmedium, haben sich Partikel mit hydrophoben Oberflächen durchgesetzt. Diese Partikel binden Proteine über hydrophobe Wechselwirkung und sind somit in der Lage, ein breites Spektrum an Peptiden bzw. Proteinen zu binden. So werden derartige magnetische Mikropartikel mit hydrophoben Oberflächen (RP-Beads) immer häufiger für die Entsalzung und Anreicherung von Peptiden und Proteinen z.B. aus Serum heraus angewendet. Diese so aus z.B. verschiedenen Seren aufgereinigten Peptide und Proteine werden nun normalerweise direkt auf ein MALDI-TOF MS Target aufgebracht, um z.B. Peptid- und Proteinprofile zu erstellen.

Serum besteht aus einer Vielzahl von Stoffen die in sehr unterschiedlichen Konzentrationen vorkommen. So kommt bspw. Albumin in einer Konzentration im mg/ml-Bereich vor, während z.B. Interleukine nur in einer Konzentration von ng/ml-Bereich vorkommt. Diese hochkonzentrierten Proteine wirken sich jedoch störend bei der Findung von niedrig konzentrierten Proteinen aus, wie sie von in einer Zellkultur wachsenden Zellen, z.B. Tumorzellen, in das Zellkulturmedium abgegeben werden. Derartige regulatorische Peptide und Proteine werden aber nur in sehr geringen Mengen abgegeben.

Hinzu kommt ferner, dass Zellen für ihr Wachstum in einem Zellkulturmedium als Zusatz im Allgemeinen noch fötales Kälberserum benötigen, kurz FKS. Dieser Zusatz liefert den Zellen zwar einerseits die notwendigen Wachstumsfaktoren, andererseits werden der Zellkultur auf diese Weise wieder zusätzliche Proteine mit sehr hohen Konzentrationen zugesetzt. Bei einer Bindung an hydrophobe Magnetbeads konkurrieren somit diese hochkonzentrierten Substanzen um die Bindungsstellen, wodurch niedrig konzentrierte Peptide und Proteine nur zu einem kleinen Bruchteil gebunden werden können.

Die mit Erfolg bei der Untersuchung von Serum bzw. Plasmaproben eingesetzten hydrophoben Magnetbeads sind somit für den Einsatz in einem Zellkulturmedium zur selektiven Separation von regulatorischen Peptiden und Proteinen, die von Tumorzellen in das Zellkulturmedium abgegeben werden, nur bedingt geeignet.

### Darstellung der Erfindung

Der Erfindung liegt die Aufgabe zugrunde ein Verfahren zur Aufreinigung wenigstens einer nachzuweisenden Zielsubstanz, die von Zellen in einem Zellkulturmedium abgegeben werden, bei dem dem Zellkulturmedium magnetische, an ihrer Oberfläche funktionalisierte Partikel, so genannte Beads, zugegeben werden, an deren Oberfläche sich die Zielsubstanz selektiv anlagert und durch Applikation eines Magnetfeldes die mit der Zielsubstanz behafteten Partikel aus dem Zellkulturmedium selektiert werden, derart weiterzubilden, dass eine selektive Separation der Zielsubstanz mit einer verbesserten Selektivität aus dem Zellkulturmedium möglich sein soll, wobei die Zielsubstanz mit nur geringer Menge bzw. Konzentration im Zellkulturmedium vorhanden ist.
Insbesondere soll das Aufreinigungsverfahren zur gezielten Extraktion von Substanzen dienen, die während der Inkubation von Zellen, insbesondere Tumorzellen, u.a. durch sekretorische Prozesse in dem Zellkulturmedium vorliegen und dies in geringer Konzentration von 10⁻¹² bis 10⁻⁷ M

Die Lösung der der Erfindung zugrunde liegenden Aufgabe ist im Anspruch 1 angegeben. Das lösungsgemäße Verfahren wird darüber hinaus durch die in den Unteransprüchen angegebenen Merkmalen sowie den in der Beschreibung insbesondere unter Bezugnahme auf die Ausführungsbeispiele entnehmbaren Merkmalen vorteilhaft weitergebildet.

Das lösungsgemäße Verfahren zur Aufreinigung wenigstens einer nachzuweisenden Zielsubstanz, vorzugsweise von regulatorischen Peptiden und Proteinen, die von Zellen, insbesondere von Tumorzellen, in einem Zellkulturmedium abgegeben werden, besteht darin, dass (a) die Zellkultur unter der Zuhilfenahme eines Serumersatzes durchgeführt wird und dass (b) dem Zellkulturmedium magnetische, an ihrer Oberfläche funktionalisierte Partikel, so genannte Beads, zugegeben werden, an deren Oberfläche sich die Zielsubstanz selektiv anlagert und durch Applikation eines Magnetfeldes die mit der Zielsubstanz behafteten Partikel aus dem Zellkulturmedium selektiert werden, zeichnet sich durch folgende Verfahrensschritte aus: (Zur besseren Verfolgbarkeit der einzelnen Hauptprozessschritte sind zu diese zugehörige Schlagworte fett editiert.)

**Bereitstellen eines Serumersatzes,** der aus einem natürlich vorkommenden Serum gewonnen wird und frei oder nahezu frei ist von niedermolekularen Substanzen, die eine Masse von kleiner gleich 60 kDa, 30 kDa, vorzugsweise kleiner gleich 10 kDa, aufweisen. So zeichnet sich der bereitzustellende Serumersatz insbesondere dadurch aus, dass er sich nicht, wie bei anderen Produkten, nachteilig auf das Zellwachstum auswirkt, aber vor allem keine störenden Substanzen mit einer Größe unterhalb 10 kDa enthält.

Als natürliches Serum bieten sich grundsätzliche tierische Seren an, vorzugsweise fötales Kälberserum, Rinder- oder Schafsserum, um nur einige zu nennen. Auch kann menschliches Serum verwendet werden.

Zur Aufbereitung und Bereitstellung des Serumersatzes wird in einer bevorzugten Ausführungsvariante das natürliche Serum zunächst unter Einsatz von Ultrafiltrationseinheiten zentrifugiert, so dass ein Überstand, das so genannte Retentat, und ein Permeat erhalten wird. Im Letzteren, dem Permeat, befinden sich je nach verwendeter Filtergröße Substanzen mit Partikelgrößen kleiner der jeweils verwendeten Filterporengröße. Hierbei kommen Membranen mit geeignet gewählten Porengrößen bzw. Ausschlussgrenzen zwischen 3 KDa und 100 kDa zum Einsatz. Um einen möglichst an Kleinstpartikeln befreiten Serumüberstand zu erhalten, wird der im Rahmen des ersten Zentrifugationsdurchgang erhaltene Überstand mit einem Nährmedium versetzt und einer erneuten Zentrifugation unterzogen. Dieser auch als Waschvorgang zu bezeichnende Vorgang wird in vorteilhafter Weise mehrmals wiederholt. Als Waschlösung, das dem jeweils erhaltenen Überstand beizumischen ist, bieten sich Nährmedien, wie Dulbecco's Modified Eagle Medium, kurz DMEM, IMDM, IMEM, ERDF, RPM11640 oder Puffer, wie PBS, MES, TRIS, etc. an.

Nach dem Waschvorgang erfolgt im Weiteren ein Rückzentrifugieren des erhaltenen Überstandes bzw. des Retentats, wobei das Gefäß in dem der Überstand enthalten ist lediglich umgedreht wird und abermals einer Zentrifugation unterzogen wird, wobei nun das Retentat aus der Zentrifugationseinheit zur weiteren Verarbeitung in ein Auffanggefäß herausbefördert und mit dem jeweils eingesetzten Nährmedium oder Puffer versetzt wird, vorzugsweise durch Zugabe von soviel an Nährmedium, dass das ursprünglich eingesetzte Volumen erreicht wird.

Der auf die vorstehende Weise erhaltene, so bezeichnete Serumüberstand wird zu Zwecken einer weiteren Reinigung sowie Sterilisation einer sterilen Filtration unterzogen, vorzugsweise unter Einsatz eines 0,2 µm-Filters, und anschließend als der dem lösungsgemäßen Verfahren bereitzustellende Serumersatz in praktikable Mengen von bspw. 500 µl-Aliquots portioniert und für eine künftige Verwendung bei - 20°C bis - 80°C eingefroren.

**Beimischen** des vorstehend beschriebenen Serumersatzes zu dem mit den Zellen versetzen Zellkulturmedium, für das gleichsam dem vorstehend beschriebenen Nährmedium Dulbecco's Modified Eagle Medium, kurz DMEM, IMDM, IMEM, ERDF oder RPMI1640 geeignet ist. Das Beimischen des Serumersatzes erfolgt mit einem Volumenanteil von 1-10%, in dem die Zellen über mehrere Tage, d.h. 1 bis 14 Tage, **inkubiert** werden. Ein während der Inkubation des Gemisches aus Zellkuturmedium, Zellen und Serumersatz mit Substanzen angereicherter Zellkulturüberstand, wird im Weiteren **separiert** und zu Zwecken eines Transportes oder zu Zwecken der Aufbewahrung in entsprechend konfektionierte Behältnisse, so genannte Vials, abgefüllt und gegebenenfalls eingefroren.

Zur weiteren Verarbeitung bzw. Aufreinigung der sich innerhalb des Zellkulturüberstandes befindlichen Substanzen wird der Zellkulturüberstand nach entsprechendem Auftauen einer **Zentrifugation** mittels einer Ultrafiltrationseinheit unterzogen. Hierbei finden Membranen mit geeignet gewählten Porengrößen bzw. Ausschlußgrenzen zwischen 3 kDa bis 60 kDa bevorzugt Einsatz. Das durch die **Filtration** gewonnene Filtrat wird im Weiteren durch Behandlung mit geeignet funktionalisierten Magnetbeads aufgereinigt.

Zur erfolgreichen Anwendung des lösungsgemäßen Verfahrens sind besonders funktionalisierte, magnetische Partikel, kurz Beads oder Magnetbeads, **bereitzustellen**, die im Vergleich zu bislang kommerziell erhältlichen Beads über eine höhere Bindekapazität und eine neue Methode zur Bindung von Substanzen verfügen, die sich durch eine hohe Anreicherung der niedrig konzentrierten Substanzen auszeichnet. So weisen die neuartigen Magnetbeads an ihren funktionalisierten Oberflächen eine Vielzahl an Dendrimeren mit jeweils bis zu 10 Verzweigungen auf, d.h. zehn Generationen, wobei die Endstellen der jeweils letzten Generation der Dendrimere modifiziert werden.

Derartig neue Magnetbeads sind beispielsweise in folgender Weise herstellbar bzw. konfektionierbar:

Zunächst gilt es die magnetischen Partikel bereitzustellen, die es kommerziell in unterschiedlichen Partikelgrößen im Nano- oder Mikro-Bereich, d.h. ein oder mehrere nm bzw. µm, zu erhalten gibt, an deren Oberflächen beispielsweise Aminogruppen appliziert sind. Zur weiteren Funktionalisierung der Aminobeadoberfläche werden die Beads in ein organisches Lösungsmittel gegeben, dem Methylacrylat zugegeben worden ist. Nach einer Einwirkzeit, die vorzugsweise 2 bis 48 Stunden von Dauer ist, werden die Beads aus der Reaktionsflüssigkeit abgetrennt und mit frischem Lösungsmittel gewaschen. Anschließend werden die so vorbereiteten Beads zur Ausbildung einer ersten Dendrimergeneration (G1-Beads) an ihrer Oberfläche in eine organische Reaktionsflüssigkeit, der Ethylendiamin zugegeben worden ist. In dieser Umgebung reagieren die Beads weitere 2 bis 48 Stunden, wobei der Reaktionsvorgang durch Schütteln des Reaktionsbades unterstützt werden kann. Anschließend werden die Beads aus der Reaktionsflüssigkeit erneut, vorzugsweise unter Verwendung eines Magneten separiert und gewaschen. Um nun eine mehrfache Dendrimerausbildung zu erlangen, auf die letztlich die überaus hohe Bindefähigkeit der neuartig funktionalisierten Beads zurückzuführen ist, gilt es den vorstehend beschriebene Vorgang zur Ausbildung der ersten Dendrimergeneration G1 bis zu neun mal zu wiederholen.

Liegen nach entsprechender Prozessierung Dendrimer-Beads vor so werden diese in einem Lösungsmittel unter Hinzufügen von aktivierten Alkyl- (C₁-C₃₀) oder Aromatenderivaten, z.B. Monoaromaten oder Polyaromaten, suspendiert, um letztlich an den Dendrimerkettenendstellen eine Modifizierung mit hydrophoben Derivaten zu erreichen, wodurch die Beads über hydrophobe Eigenschaften verfügen. Man bezeichnet diese funktionalisierten Beads deshalb auch als dendritische Reversed Phase Beads (G1-RP-Beads bis G10-RP-Beads).

Zur Bereitstellung werden die Beads aus der Reaktionssuspension separiert, abermals gewaschen und in destilliertem Wasser oder Puffer, wie PBS, MES, TRIS etc. bevorratet.

Die auf die vorstehende Weise erhaltenen, so genannten Dendrimer-RP-Beads werden zu Zwecken der weiteren Aufreinigung und Separierung der sich innerhalb des steril gefilterten Zellkulturüberstandes befindlichen Substanzen, u.a. von den Zellen sekretierte regulatorische Peptide und Proteine dem nach der Filtration erhaltenen Filtrats unter Zugabe einer Pufferlösung, **beigemischt**. Anschließend erfolgt eine **Inkubation** mit einer Verweilzeit von 10 bis 120 Minuten bei Raumtemperatur. Hierbei erfolgt die Bindung der in dem Zellkulturüberstand befindlichen Substanzen an die modifizierten Endstellen der Dendrimerstrukturen. Anschließend erfolgt die **magnetische Selektion** der magnetischen Beads aus dem Gemisch und eine **Elution** der an der dendritischen Struktur gebundenen nachzuweisenden Substanzen, vornehmlich die von den Zellen sekretierten Substanzen, allen voran die regulatorischen Peptide und Proteine. Selbstverständlich können je nach Funktionalisierung der Magnetbeads auch weitere Substanzen gezielt selektiert werden, so bspw. Metabolite, Steroide etc.

Der Kerngedanke des lösungsgemäßen Aufreinigungsverfahren umfasst im Wesentlichen zwei vorteilhaft zusammenwirkende Grundgedanken, nämlich zum einen die Bereitstellung eines Zellkulturzusatzes bzw. Serumersatzes, in dem von vornherein keine störende Kleinsubstanzen enthalten sind, die möglicherweise bei der nachfolgenden Analyse in Konkurrenz mit den eigentlich nachzuweisenden Zielsubstanzen treten, zum anderen neuartiger Magnetbeads mit einer dendritischen Struktur und einer RP-Phase (reversed phase), wobei die dendritische Struktur bis zu zehn Verzweigungsstellen aufweist und somit die Anzahl der für eine Bindung von nachzuweisenden Zielsubstanzen optimiert.

Durch das lösungsgemäße Verfahren können weit mehr Substanzen isoliert werden als bei der Verwendung von bisher im Einsatz befindlichem normalem Serum, wie bspw. fötales Kälberserum. Durch Verwendung der neuartigen Beads kann im Vergleich zu kommerziell erhältlichen eine 10 bis 100-fache Steigerung der Sensitivität aus bsp. Zellkulturübertständen erreicht werden. Zusammengefasst wird bei der Verwendung der Beads und des Serumersatzes eine bis zu 1000-fach höhere Empfindlichkeit erzielt, wie bei der Anwendung von normalem Serum und kommerziell erhältlichen Beads.

Im Weiteren werden einige konkrete Ausführungsbeispiele zur Verfahrensführung dargestellt:

### Beispiel 1: Herstellung eines Serumersatzes

(a) Niedermolekulare Substanzen aus fötalem Kälberserum oder aus humanem Serum werden durch Zentrifugation und Waschen mit Dulbeccos Eagle Modified Medium entfernt. Hierbei erfolgt die Zentrifugation über eine Filtrationsmembran, die je nach Bedarf eine Porengröße zwischen 3 kDa und 100 kDa haben kann. Der Zentrifugationsvorgang wird eine Stunde durchgeführt. Bei Bedarf kann das Serum mit Proteaseinhibitoren (PMSF, EDTA, Proteaseinhibitoren in Form von einzelnen Inhibitoren oder eines Cocktails etc.) vor der Aufarbeitung versetzt werden.
(b) Der im Wege der Zentrifugation erhaltene Überstand wird mit Dulbeccos Medium Eagle Medium (DMEM) versetzt und wieder 1 Stunde zentrifugiert (Reinigung des Serums).
(c) Der Zentrifugations- und Waschschritt werden insgesamt 2-5x durchgeführt.
(d) Der erhaltene Überstand wird nun in ein Gefäß rückzentrifugiert und mit DMEM auf das ursprünglich eingesetzte Volumen eingestellt.
(e) Der erhaltene Serumersatz (auch Serumüberstand genannt) wird über einen 0.2µm-Filter steril filtriert.
(f) Der Serumersatz wird zu 500 µl-Aliquots portioniert und bei -20°C bis zur Verwendung eingefroren.

### Beispiel 2: Herstellung von modifizierten Magnetbeads

Ausgehend von der Aminoberfläche der Beads wird über eine Verzweigungsreaktion an jeder Aminogruppe eine dendritische Struktur aufgebaut. Hierbei wird das Prinzip zur Synthese von Starburstmolekülen zugrunde gelegt, bis eine bis zu zehnfache-Verzweigung in der dendritischen Struktur zum Erhalt so genannter G1- bis G10-Beads erreicht wird. Am Schluss erfolgt die Modifizierung der endständigen NH₂-Gruppen der Dendrimer-Beads u. a. mittels Mikrowellensynthese mit unterschiedlich langen Alkylketten (C₁-C₃₀) über eine aktivierte Gruppe.
(a) Bereitstellen von 10 -2000 mg Aminobeads und in einem Behältnis mehrmals mit 10 bis 100 ml MeOH waschen.
(b) Beads in 10 -100 ml MeOH aufnehmen und 10- 100 ml kaltes Methylacrylat zugegeben. Auf einem Schüttler 2-48 Stunden bei Raumtemperatur (RT) reagieren lassen.
(c) Abtrennen der Beads von der Reaktionsflüssigkeit durch einen Magneten und Beads 3x mit jeweils 10- 200 ml MeOH waschen.
(d) Zugabe von 10 - 100 ml MeOH und langsame Zugabe von 10 -100 ml kaltem Ethylendiamin. Bei Raumtemperatur 2 - 48 h unter Schütteln regieren lassen; (G1 -Beads).
(e) siehe Schritt (c), d.h. Abtrennen der Beads von der Reaktionsflüssigkeit durch einen Magneten und Beads 3x mit jeweils 10 -100 ml MeOH waschen.
(f) N-malige Wiederholung der Schritte (b) - (e), wobei n= 0 bis 9 ist.
(g) Waschen der Dendrimer-Beads jeweils 3x mit 10-100 ml MeOH und 5x mit 10- 100 ml halogeniertem Kohlenwasserstoff,
(h) Dendrimer-Beads in 50-100 ml halogeniertem Lösungsmittel suspendieren und langsames Zutropfen von 5 -10 ml eines aktivierten Alkylderivates, z.B. C1-C30 oder Aromatenderivates. Reaktion-für 10 bis 120 Minuten bei RT im Ultraschallbad und/oder unter Erhitzen bis maximal 38°C oder mittels Mikrowellensynthese.
(i) Modifizierte Dendrimer-Beads jeweils 5 x mit 10 - 100 ml MeOH und 5 x mit 10 -100 ml ultrareinem Wasser waschen.
(j) Aufnahme der Dendrimer-Beads in ultrareinem Wasser und Lagerung bei +4°C.

Alternativ ist es anstelle des Verfahrensschrittes (h) ebenso möglich die Dendrimer-Beads anderweitig an den Endstellen ihrer Dendrimerketten zu modifizieren, bspw. durch Hinzufügung von Antikörpern. Hierzu werden die vorprozessierten dendritischen Aminobeads (G1-G10) in einem Puffer mit einem bifunktionalem Linker für Aminogruppen zur Reaktion gebracht. Als Linker eignet sich vorzugsweise Glutardialdehyd, Bis (Sulfosuccinimidyl) suberate, Succinimidylsuberate, Dimethyl Pimelidate oder bifunktionelle PEG-Linker. Anschließend erfolgt ein Waschen der Beads vorzugsweise in einem Phosphatpuffer. Schließlich werden dem Puffer Antikörper in einer Konzentration von 1 mg/ml bis 50 mg/ml Puffer zugegeben. Nach einer Inkubation von ca. 1 -14 h bei RT oder +4°C werden die mit Antikörpern versehenen Beads gewaschen und in einem Puffer zur weiteren Verwendung bei etwa +4°C bevorratet.

### Beispiel 3: Durchführung einer Aufreinigung von sekretierten Substanzen von in einem Zellkulturmedium vorliegenden Tumorzellen unter Verwendung des in Beispiel 1 gewonnenen Serumersatzes sowie der in Beispiel 2 modifizierten magnetischen Partikel

Die Tumorzellen werden je nach Größe des Experiments in Dulbeccos Modified Eagle Medium (DMEM) und 1-10% Serumersatz, der aus fötalem Kälberserum (FKS) erhalten worden ist, in verschiedenen Behältnissen eingesät und über mehrere Tage bei 37°C und 5% CO₂-Anreicherug inkubiert. Der Zellkulturüberstand, d.h. DMEM + 1-10% FKS-Serumersatz, kurz ZKÜ genannt, wird in vorher behandelte Vials abgefüllt und zum Transport bei -20°C eingefroren.

Vor der Aufreinigung mit den modifizierten Dendrimer-Beads (G1-G10) wird der aufgetaute ZKÜ über eine Membranfiltrationseinheit filtriert. Das Filtrat wird im Weiteren mittels der modifizierten Dendrimer-Beads (G1-G10) aufgereinigt und konzentriert. Anschließend erfolgt die Elution und weitere Aufarbeitung.

Im Einzelnen:
(a) Zellen in DMEM und FKS-Überstand aufnehmen und je nach Ansatz (Volumen) in geeignetem Zellkulturgefäß bei 37°C und 5% CO₂ für 3 Tage inkubieren.
(b) Verwerfen des ZKÜ und vorsichtiges Waschen der Zellen mit jeweils 10 ml PBS, bei pH 7.2 oder DMEM, den Waschvorgang 2-5x wiederholen.
(c) Zugabe der erforderlichen Menge DMEM + 1-10% FKS-Serumüberstand und Inkubation der Zellen über die erforderliche Zeit (1- 14 Tage). Nach erfolgter Inkubation ZKÜ's in vorbehandelte Vials (1 ml) geben und bei -20°C oder bei -80°C einfrieren.
(d) Auftauen der eingefroren ZKÜ's und jeweils 500 µl in eine Filtrationseinheit überführen.
(e) Zentrifugation der ZKÜ für 1 h bei 12000 rpm und Filtrat auffangen.
(f) In der Zwischenzeit Vorbereitung der Beads für die Inkubation. 80 µl der modifizierten Dendrimer-Beads (G1- G10) in 450 µl Puffer bei pH 7.2 aufnehmen und kurz suspendieren. Danach Abtrennen der Beads über einen Magneten.
(g) Zugabe von 450 µl eines Puffers mit pH 7.2 und Beads suspendieren. Anschließend 450 µl des von der ZKÜ-Zentrifugation efialtenen Filtrats mit einer Pipette zugeben und 10 x auf und ab pipettieren (Beads vollständig suspendieren).
(h) Inkubation für 10 - 120 min bei Raumtemperatur.
(i) Lösung nach magnetischer Separation verwerfen und magnetische Dendrimer-Beads (G1-G10) 3 x mit jeweils 500 µl Puffer waschen.
(j) Zugabe von 20 µl Acetonitril:TFA 1:1, v:v und mischen der Beadlösung durch 10-maliges auf- und abpipettieren (Elution).
(k) Inkubation für 1-120 min bei Raumtemperatur.
(I) Abtrennung der magnetischen Partikel und Elutionslösung für weitere Aufarbeitung separieren.

### Beispiel 4: Fünf alternative Beispiele zur weiteren Untersuchung der mit den Zielsubstanzen versehenen magnetischen Beads, wie sie nach dem lösungsgemäßen Aufreinigungsverfahren gewonnen werden.

### Erste Alternative:

Die mit den Zielsubstanzen beaufschlagten magnetischen Beads werden zu einem nicht reduzierenden oder reduzierenden Gelelektrophoreseprobenpuffer, bestehend aus TRIS, Harnstoff. Thioharnstoff, LDS, SDS oder CHAPS etc., zugegeben. Das sich dabei bildende Gemisch wird vorzugsweise auf 60°C für 5-30 min erhitzt, wodurch sich die Zielsubstanzen von den Beads lösen. Im Weiteren werden die magnetischen Beads mittels eines Magneten aus dem Gemisch abgetrennt und der verbleibende Gelelektrophoreseprobenpuffer wird mit den Zielsubstanzen abpipettiert und in Geltaschen eines Elektrophoresegels (SDS-Gel, Native-Gel, 2-D-Gel) aufgegeben und nachfolgend einer elektrophoretischen Auftrennung unterzogen.

### Zweite Alternative (gilt nur für mit Antikörper gekoppelten Dendrimer-Beads (G1-G10):

Die an den Antikörperpartikeln gebundenen Zielsubstanzen werden durch Zugabe einer Carbonsäure oder eines chaotropen Hochsalzpuffers eluiert. Das sich im Wege der Elution ergebende Carbonsäureeluat wird einrotiert (Speed-Vac) und in einer Pufferlösung aufgenommen. Schließlich unterliegen die in der Pufferlösung enthaltenen Zielsubstanzen einem enzymatischen Verdau. Die verdauten Zielsubstanzen werden anschließend einer massenspektrometrischen Identifizierung über Peptide Mass Fingerprint (PMF) oder MS/MS-Spektren unterzogen.

### Dritte Alternative:

Die mit einer oder mehreren Zielsubstanzen beaufschlagten magnetischen Beads werden gewaschen und anschließend einer Pufferlösung mit einer Protease zum Verdau der Zielsubstanzen zugeführt. Die verdauten Zielsubstanzen werden anschließend einer massenspektrometrischen Identifizierung über Peptide Mass Fingerprint (PMF) oder MS/MS-Spektren unterzogen. Desweiteren werden die magnetischen Beads abermals gewaschen und anschließend einem Gemisch aus einem organischen Lösungsmittel und/oder Carbonsäuren zugegeben, zum Erhalt eines Eluats. Letztlich wird das Eluat wieder einer massenspektrometrischen Messung unterzogen.

### Vierte Alternative:

Die an den Antikörpern gebundenen Zielsubstanzen werden durch Zugabe einer Carbonsäure oder eines Hochsalzpuffers eluiert. Zu den Eluaten werden die dendritischen reversed-phase Beads gegeben. Nach einer Inkubation von 1-60 Minuten, werden die Beads mit Hilfe eines Magneten separiert und anschließend mehrmals mit einem Puffer gewaschen.

Anschließend erfolgt entweder der in Alternative 1 aufgezeigte Weg für eine Gelelektrophoretische Auftrennung oder der in Alternative 3 aufgezeigte Weg für eine Erzeugung von proteolytischen Peptide, die anschließend über MS- oder MS/MS-Spektren detektiert und aufgeklärt werden.

### Fünfte Alternative:

Zellen, wie z.B. Tumorzellen, werden je nach Experiment in verschiedene Behältnisse eingesät und für mehrere Tage bei 37°C und 5% CO₂-Gehalt inkubiert. Danach erfolgt ein Waschen der Zellen mit Zellkulturmedium oder Puffer. Anschließend erfolgt die Zugabe von Zellkulturmedium ohne Serum und eine nochmalige Inkubation von 0 - 72 h. Die jeweiligen Zellkulturüberstände werden abgefüllt und gegebenenfalls bei -20°C eingefroren.

Die Bindung der Substanzen an die modifizierten Dendrimerbeads erfolgt durch Zugabe derselben zu den aufgetauten Zellkulturüberständen . Anschließend erfolgt ein Waschen der Beads und diese werden wie in Alternative 1 aufgezeigt, einer Gelelektrophorese unterzogen oder wie in Alternative 3 aufgezeigt einem Verdau mit einer Protease unterzogen und die verdauten Substanzen einer MS bzw. MS/MS-Analyse unterzogen.

Die vorstehend beschriebenen funktionalisieren Magnetbeads stellen als solche ein Produkt dar, das als sensorisch wirkende Substanz grundsätzlich für eine Vielzahl unterschiedlicher Einsatzwecke verwendbar ist. Ein möglicher Einsatz ist die vorstehend beschriebene Aufreinigung von Zielsubstanzen. Zur Herstellung eines solchen Produktes in allgemeinen Form sind folgende Maßnahmen zu treffen:
a) Bereitstellen von magnetischen Partikeln, kurz Beads, mit einer Partikelgröße im Nano- und/oder Mikro-Bereich, d.h. ein oder mehrere nm oder µm, und mit an deren Oberflächen applizierte funktionelle Gruppe, wie z.B. Aminogruppen,
b) Reagieren der Beads in einem Verzweigungsreagenz, wie z.B.
   Methylacrylat enthaltenden organischen Lösungsmittel,
c) Abtrennen der Beads aus dem organischen Lösungsmittel und Waschen der Beads mit einem organischen Lösungsmittel,
d) Reagieren der Beads mit einem Verbinder, wie z.B. Ethylendiamin, enthaltenden organischen Flüssigkeit,
e) Abtrennen der Beads aus der organischen Flüssigkeit und Waschen der Beads,
f) n - maliges Wiederholen der Schritte b) bis e)
g) Suspendieren der Beads in einer mit wenigstens einem Derivat enthaltenden Lösung, und
h) Abtrennen und Waschen der Beads und Bereitstellen der derivatisierten Beads in Wasser oder pufferhaltiger Lösung.

Vorzugsweise wird n = 0 bis 9 gewählt. Als Reaktionsflüssigkeit eignet sich vor allem Alkohol. Damit die Beads reversed-phase Eigenschaften annehmen, werden die Beads mit einer chemischen oder proteinogenen Gruppe, wie Alkyl-(C₁-C₃₀) oder Aromatgruppen derivatisiert. Als Grundkörper für die Beads eignen sich stabile magnetische Partikel, die eine Partikelgröße von 10 nm - 10 µm aufweisen.

Die auf den magnetischen Partikeln zu Zwecken ihrer Funktionalisierung applizierte Gruppe ist vorzugsweise aus wenigstens einem Stoff der nachfolgenden Stoffgruppen aufgebaut: Primäre und sekundäre Amino-, prim., sek. und unsym. Hydrazin-, Azid-, Phosphin-, Aldehyd-, Polyaldeyhd-, Carbonsäure-, Carbonsäureester-, Cyanat-, Isocyanat-, Thiocyanat-, Hydroxy-, Thiol-, Imino-, Hydrazid-, Piperidin-, Azomethin-, Semicarbazon-, Hydrazon-, Cyanbromid-, Tosyl-, Epoxid-,Cyanursäurechlorid-, Cyanursäureester,-gruppen. Diamino-, Triamino-,Tetraaminoheterocyclen.

Die vorstehend beschriebenen funktionalisierten Beads werden ferner mit Verzweigungsreagenzien, die in einem Lösungsmittel bereitgestellt werden, zur Reaktion gebracht, wobei die Verzweigungsreagenzien aus wenigstens eine der nachfolgenden Gruppe ausgewählt sind:

Melamin, Diamino-, Triamino-, Tetraaminoheterocyclen, Triepoxide, Tetraepoxide, Diallylamine, Methylacrylate, Triacrylate, Tetraacrylate, Tris(hydroxymethylamin), Oxazine, Oxetane, Diethanolamine.

Anschließend werden die mit dem Verzweigungsreagenz beaufschlagten magnetischen Partikel mit einem bi- oder trifunktionalen Linker zur Reaktion gebracht werden, wobei der der Linker aus einer der nachfolgenden Stoffzusammenstellung ausgewählt wird: Lysine, Diaminoalkane, Diamino-, Triamino-, Tetraaminoheterocyclen, Diaminoethylenglycole, Piperazin, Allylamine, Triepoxide, Tetraepoxide, Tris(hydroxymethylamin).

Schließlich werden nach n-maligem Wiederholen der vorstehend genannten Verfahrensschritte b) bis e) die magnetischen Partikel mit nachfolgenden chemischen Gruppen als Endgruppe oder mit Peptiden, Proteinen, Glycanen oder Glycoproteinen aller Art derivatisiert:
n-Alkyl-, sek-Alkyl-, tert-Alkylgruppen, unsubstituirte und substituirte Aryl-, unsubstituirte und substituirte Acyl-, primäre, sekundäre und tertiäre Amino-, prim., sek. und unsym. Hydrazin-, Azid-, Aldehyd-, Phosphin-, Polyaldeyhd-, Carbonsäure-, Carbonsäureester-, Carbonsäurehalogenid-, Carbonsäureanhydrid-, Cyanat-, Isocyanat-, Thiocyanat-, Hydroxy-, Thiol-, Sulfid-, Sulfit-, Sulfat-, Imino-, Hydrazid-, Piperidin-, Azomethin-, Semicarbazon-, Hydrazon-, Hydroxamsäure-, Amidrazon-, Amidin-, Cyanbromid-, Tosyl-, Epoxid-,Cyanursäurechlorid-Cyanursäureester-gruppen, unsubstituierte und substituierte Carbodiimide, N-Hydroxysuccinimidester, Ethylenglycole, unsubstituierte und substituierte Arylalkane, Arylalkene, Heterocyclische Verbindungen, Mono- und Polycyclische Verbindungen, Die Anzahl der Kohlenstoffe beträgt hierbei 2 bis 100.

## Patentansprüche

1. Produkt, mit Magnetbeads, die eine funktionalisierte Oberfläche aufweisen und in folgender Weise herstellbar sind:
a) Bereitstellen von magnetischen Partikeln, kurz Beads, mit einer Partikelgröße im Nano- und/oder Mikro-Bereich, d.h. ein oder mehrere nm oder µm, und mit an deren Oberflächen applizierte funktionelle Gruppe, wie z.B. Aminogruppen,
b) Reagieren der Beads in einem Verzweigungsreagenz, wie z.B. Methylacrylat enthaltenden organischen Lösungsmittel,
c) Abtrennen der Beads aus dem organischen Lösungsmittel und Waschen der Beads mit einem organischen Lösungsmittel,
d) Reagieren der Beads mit einem Verbinder, wie z.B. Ethylendiamin, enthaltenden organischen Flüssigkeit,
e) Abtrennen der Beads aus der organischen Flüssigkeit und Waschen der Beads,
f) n - maliges Wiederholen der Schritte b) bis e)
g) Suspendieren der Beads in einer mit wenigstens einem Derivat enthaltenden Lösung, und
h) Abtrennen und Waschen der Beads und Bereitstellen der derivatisierten Beads in Wasser oder pufferhaltiger Lösung.

2. Produkt nach Anspruch 1,
**dadurch gekennzeichnet, dass** als n = 0 bis 9 gewählt wird.

3. Produkt nach Anspruch 1 oder 2,
**dadurch gekennzeichnet, dass** als Reaktionsflüssigkeit Alkohol verwendet wird.

4. Produkt nach einem der Ansprüche 1 bis 3,
**dadurch gekennzeichnet**, das die Beads mit einer chemischen oder proteinogenen Gruppe, wie z.B. Alkyl-(C₁-C₃₀) oder Aromatgruppen derivatisiert werden und somit z.B. reversed-phase Eigenschaften annehmen.

5. Produkt nach einem der Ansprüche 1 bis 4,
**dadurch gekennzeichnet, dass** als Grundkörper stabile magnetische Partikel verwendet werden, die eine Partikelgröße von 10 nm - 10 µm aufweisen.

6. Produkt nach einem der Ansprüche 1 bis 5,
**dadurch gekennzeichnet, dass** die auf den magnetischen Partikeln applizierte Gruppe beispielsweise aus einem Stoff der nachfolgenden Stoffgruppen aufgebaut ist:
Primäre und sekundäre Amino-, prim., sek. und unsym. Hydrazin-, Azid-, Phosphin-, Aldehyd-, Polyaldeyhd-, Carbonsäure-, Carbonsäureester-, Cyanat-, Isocyanat-, Thiocyanat-, Hydroxy-, Thiol-, Imino-, Hydrazid-, Piperidin-, Azomethin-, Semicarbazon-, Hydrazon-, Cyanbromid-, Tosyl-, Epoxid-,Cyanursäurechlorid-, Cyanursäureester,-gruppen, Diamino-, Triamino-,Tetraaminoheterocyclen.

7. Produkt nach Anspruch 6,
**dadurch gekennzeichnet, dass** die funktionalisierten Beads mit Verzweigungsreagenzien, die in einem Lösungsmittel bereitgestellt werden, zur Reaktion gebracht werden und dass die Verzweigungsreagenzien aus wenigstens eine der nachfolgenden Gruppe ausgewählt sind:
Melamin, Diamino-, Triamino-, Tetraaminoheterocyclen, Triepoxide, Tetraepoxide, Diallylamine, Methylacrylate, Triacrylate, Tetraacrylate, Tris(hydroxymethylamin), Oxazine, Oxetane, Diethanolamine.

8. Produkt nach einem der Ansprüche 1 bis 7,
**dadurch gekennzeichnet**, das die mit dem Verzweigungsreagenz beaufschlagten magnetischen Partikel mit einem bi- oder trifunktionalen Linker zur Reaktion gebracht werden, und dass der Linker aus der nachfolgenden Stoffzusammenstellung ausgewählt wird:
Lysine, Diaminoalkane, Diamino-, Triamino-, Tetraaminoheterocyclen, Diaminoethylenglycole, Piperazin, Allylamine, Triepoxide, Tetraepoxide, Tris(hydroxymethylamin).

9. Produkt nach einem der Ansprüche 1 bis 8,
**dadurch gekennzeichnet, dass** nach n-maligem Wiederholen der Schritte b) bis e) die magnetischen Partikel mit nachfolgenden chemischen Gruppen als Endgruppe oder mit Peptiden, Proteinen, Glycanen oder Glycoproteinen aller Art derivatisiert werden:
n-Alkyl-, sek-Alkyl-, tert-Alkylgruppen, unsubstituirte und substituirte Aryl-, unsubstituirte und substituirte Acyl-, primäre, sekundäre und tertiäre Amino-, prim., sek. und unsym. Hydrazin-, Azid-, Aldehyd-, Phosphin-, Polyaldeyhd-, Carbonsäure-, Carbonsäureester-, Carbonsäurehalogenid-, Carbonsäureanhydrid-, Cyanat-, Isocyanat-, Thiocyanat-, Hydroxy-, Thiol-, Sulfid-, Sulfit-, Sulfat-, Imino-, Hydrazid-, Piperidin-, Azomethin-, Semicarbazon-, Hydrazon-, Hydroxamsäure-, Amidrazon-, Amidin-, Cyanbromid-, Tosyl-, Epoxid-,Cyanursäurechlorid-Cyanursäureester-gruppen, unsubstituierte und substituierte Carbodiimide, N-Hydroxysuccinimidester, Ethylenglycole, unsubstituierte und substituierte Arylalkane, Arylalkene, Heterocyclische Verbindungen, Mono- und Polycyclische Verbindungen.
